# EUROPEAN PATENT APPLICATION

(11) **EP 4 653 530 A1**
(43) Date of publication of application: **26.11.2025**
(21) Application number: 24382561.9
(22) Date of filing: 24.05.2024
(51) Int. Cl.: C12N 15/113

(54) **ARTIFICIAL RIBOZYMES AND USES THEREOF**

(71) Applicant: Nocturna Therapeutics SL, 08018 Barcelona (ES)
(72) Inventor: Dotu Rodriguez, Ivan Javier, 08018 Barcelona (ES); Talló Parra, Marc, 08018 Barcelona (ES)
(74) Representative: Hoffmann Eitle

(57) **Abstract**

The present invention provides synthetic catalytic polyribonucleotide comprising SEQ ID NO: 33, or a system of molecules that give rise to said synthetic catalytic polyribonucleotide, wherein the catalytic polyribonucleotide is capable of exerting a catalytic cleavage activity on a substrate polyribonucleotide, wherein the substrate polyribonucleotide compris-es the sequence of SEQ ID NO: 20, and wherein N₁, N₂, N₃, and N₄ can be any nucleotides, provided that N₁ is capable of base pair with N₄ and N₂ is capable to base pair with N₃.

## Description

### TECHNICAL FIELD

The present invention relates to the field of molecular biology. Particularly, the present invention relates to the field of artificial ribozymes.

### BACKGROUND ART

Over the past three decades, it has become evident that RNA and DNA can catalyze a broad range of chemical reactions. Nucleic acids can fold into complex three-dimensional structures including binding sites and catalytic centers and provide an environment in which various reactions can be facilitated. Extensive research in this field has shown that RNA and DNA are able to accelerate the formation, cleavage and rearrangement of various types of covalent bonds.

A catalytic ribozyme refers to an RNA having an enzyme activity (catalytic ability), i.e., an RNA catalyst. The term ribozyme is a fusion of two words, ribonucleic acid (RNA) and enzyme. The so-called "hammerhead" structures are a class of naturally occurring RNAs structures which undergo site specific autolytic cleavage, as they consist of a small catalytic RNA motif capable of endonucleolytic (self-) cleavage. The majority of these self-cleaving RNAs originates from satellite RNAs of plant viruses, and they are composed of a catalytic core of conserved nucleotides flanked by three helices, two of which form essential tertiary interactions for fast self-scission under physiological conditions. Its presence in several eukaryotic genomes has been widely reported since their discovery.

Using two synthetic RNA oligomers which can together form a hammerhead structure, it was demonstrated that cleavage can occur in trans, i.e. that one molecule can catalyze the cleavage of the other. When cleavage occurs in trans, the RNA which can be cleaved, is considered the substrate (target RNA) and the RNA which catalyzes the cleavage, is designated "ribozyme". Ribozymes assuming a hammerhead structure are designated "hammerhead ribozymes".

Currently, natural ribozymes are being developed as treatments for a variety of diseases ranging from inborn metabolic disorders to viral infections and acquired diseases such as cancer. Ribozymes can be used both to down-regulate and to repair pathogenic genes. However, although some variations on naturally occurring ribozymes are available, they have not been very effective in mammalian cells.

Thus, there is a need to develop more efficient ribozymes that show improved activity and function in mammalian cells with high efficiency. These ribozymes will useful for developing regulated gene expression systems and have great therapeutic values.

### BRIEF DESCRIPTION OF DRAWINGS

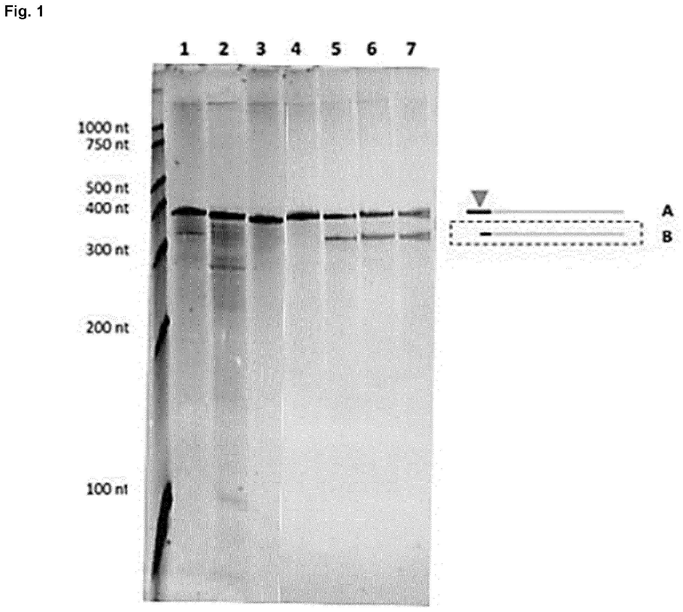

**Fig. 1****:** Self-processing of 5' HH ribozymes of the RNA constructs transcribed from linearized plasmids (7% polyacrylamide-urea gel electrophoresis). The transcription reaction generates a full-length transcript (A) and a shorter RNA entity of approximately 300 nt (B) which corresponds to the self-processed RNA by the 5' HH ribozyme. Each lane corresponds to a different 5'HH: Ricordea florida (SEQ ID NO 1), Trichobilharzia regenti (SEQ ID NO 2), Schistosoma rodhaini (SEQ ID NO 3-4), and our artificial designed ribozymes (SEQ ID NO 5-7).

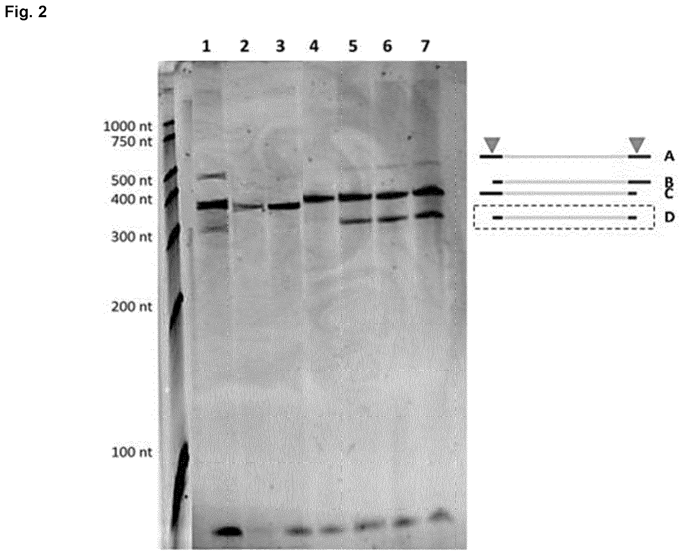

**Fig. 2****:** Self-processing of 5' and 3' HH of the RNA transcribed from linearized plasmids (7% polyacrylamide-urea gel electrophoresis). The transcription reaction generates a non-cleaved full-length transcript (A) as well as two shorter RNA entities (B-C) where one of the ribozymes is not cleaved. The 300 nt band (D) is the one which corresponds to the self-processed circRNA by the two HH ribozymes. All constructs contain a different 5'HH and the ELVd ribozyme in the 3' region. Each lane corresponds to a different 5'HH: Ricordea florida (SEQ ID NO 12), Trichobilharzia regenti (SEQ ID NO 13), Schistosoma rodhaini (SEQ ID NO 14-15), and our artificial designed ribozymes (SEQ ID NO 16-18).

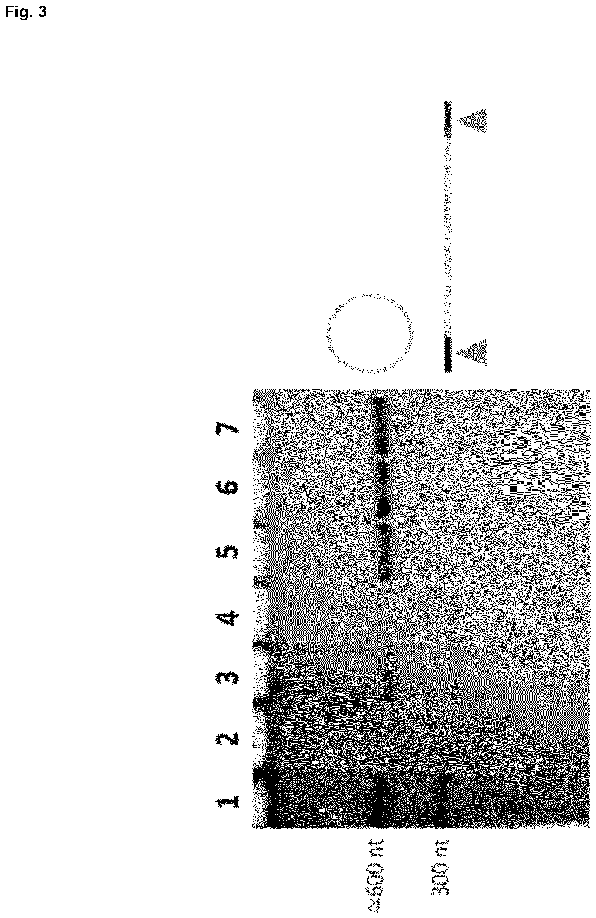

**Fig. 3****:** Circularization efficiency of 7 precursor circRNAs from Example 2. The circularization reaction generates one band corresponding to circular RNA molecules (around 600 nt) and in some cases a second band (around 300 nt) corresponding to the linear RNA not circularized. Each lane corresponds to the circularization of one precursor generated with a different 5'HH in this order: Ricordea florida (SEQ ID NO 12), Trichobilharzia regenti (SEQ ID NO 13), Schistosoma rodhaini (SEQ ID NO 14-15), and our artificial designed ribozymes (SEQ ID NO 16-18).

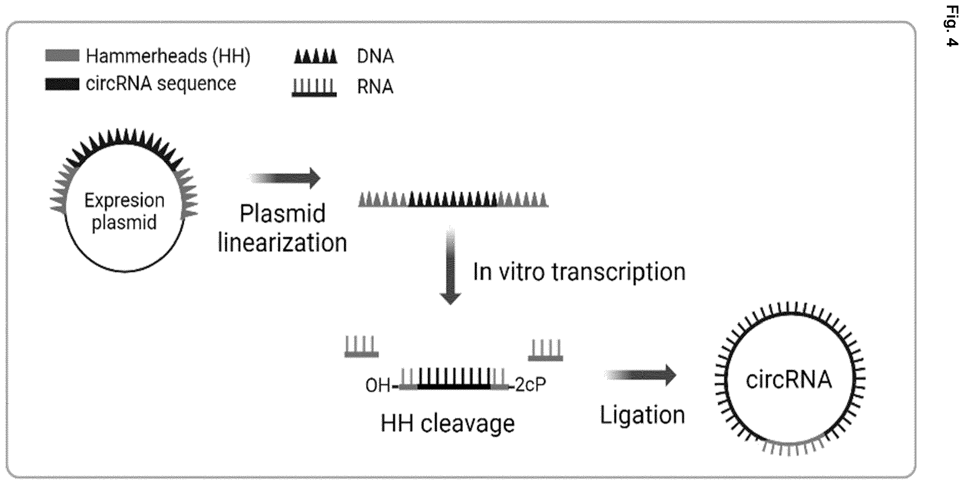

**Fig. 4****:** Schematic synthesis of the circular RNA. CircRNA sequences of interest are cloned in an expression plasmid flanked by hammerheads (HH). After plasmid linearization, an in vitro transcription step is performed where the HH autocleave themselves leaving an hidroxyl end group (OH) in the 5' and a 2'-3'-cyclophosphate end group (2cP) in the 3' of the linear RNA. After a ligation reaction, circRNAs are produced.

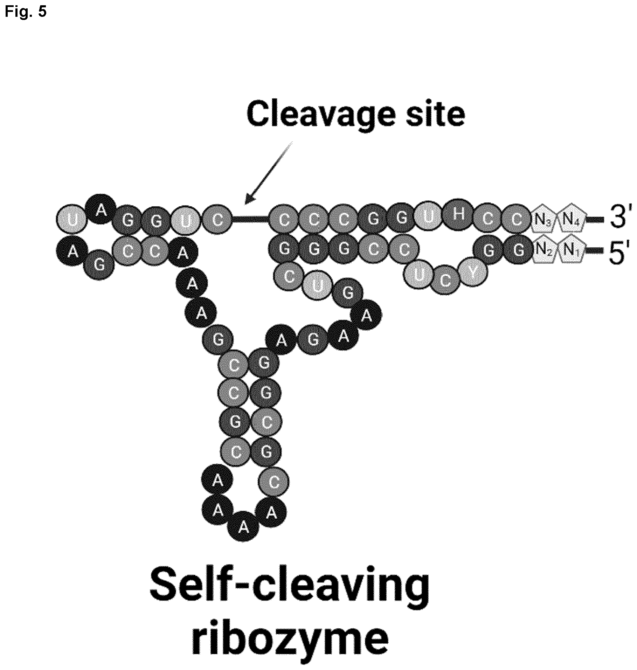

**Fig. 5****:** Schematic representation of self-cleaving ribozyme before cleavage.

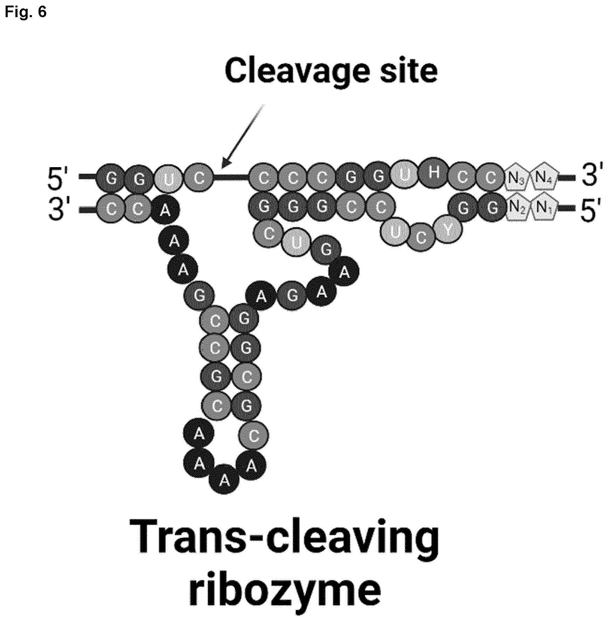

**Fig. 6****:** Schematic representation of trans-cleaving ribozyme.

### SUMMARY OF THE INVENTION

The present invention provides:
1. A synthetic catalytic polyribonucleotide comprising SEQ ID NO: 33, or a system of molecules that give rise to said synthetic catalytic polyribonucleotide,
   wherein the catalytic polyribonucleotide is capable of exerting a catalytic cleavage activity on a substrate polyribonucleotide, wherein the substrate polyribonucleotide comprises the sequence of SEQ ID NO: 20, and wherein N₁, N₂ in SEQ ID NO: 33, and N₃, N₄ in SEQ ID NO: 20 can be any nucleotide, provided that N₁ is capable of base pair with N₄ and N₂ is capable to base pair with N₃.
2. The synthetic catalytic polyribonucleotide according to 1, wherein:
   a) the polyribonucleotide comprises or consists of SEQ ID NO: 23, wherein the polyribonucleotide is capable of exerting a catalytic cleavage activity on a substrate polyribonucleotide, wherein the substrate polyribonucleotide preferably comprises the sequence of SEQ ID NO: 24;
   b) the polyribonucleotide comprises or consists of SEQ ID NO: 25, wherein the polyribonucleotide is capable of exerting a catalytic cleavage activity on a substrate polyribonucleotide, wherein the substrate polyribonucleotide preferably comprises the sequence of SEQ ID NO: 26; or
   c) the polyribonucleotide comprises or consists of SEQ ID NO: 27, wherein the polyribonucleotide is capable of exerting a catalytic cleavage activity on a substrate polyribonucleotide, wherein the substrate polyribonucleotide preferably comprises the sequence of SEQ ID NO: 28.
3. The synthetic catalytic polyribonucleotide according to any one 1 or 2, wherein the substrate polyribonucleotide is comprised in the transcriptome of an organism and wherein the catalytic polyribonucleotide is not comprised in the transcriptome of said organism, so that the catalytic polyribonucleotide exerts a *trans* catalytic cleavage activity on said substrate polyribonucleotide.
4. A synthetic self-catalytic polyribonucleotide, or a system of molecules that give rise to said synthetic self-catalytic catalytic polyribonucleotide, comprising at least two regions are covalently connected by a linker, wherein a first region comprises the synthetic catalytic polyribonucleotide as defined in any of 1 to 3, wherein a second region comprises the substrate polyribonucleotide of said synthetic catalytic polyribonucleotide, and wherein the catalytic polyribonucleotide of the first region exerts a *cis* catalytic cleavage activity on the substrate polyribonucleotide of the second region.
5. The synthetic self-catalytic polyribonucleotide according to 4, wherein the linker is preferably the nucleotide sequence "GAUA".
6. The synthetic self-catalytic polyribonucleotide according to 5, comprising any of the following sequences:
   a) SEQ ID NO: 21, wherein N₁, N₂, N₃, and N₄ can be any nucleotides, provided that N₁ is capable of base pair with N₄ and N₂ is capable of base pair with N₃, and wherein N₅ is a linker, preferably the nucleotide sequence "GAUA";
   b) SEQ ID NO: 22, wherein N₁, N₂, N₃, and N₄ can be any nucleotides, provided that N₁ is complementary to N₄ and N₂ is complementary to N₃;
   c) SEQ ID NO: 29;
   d) SEQ ID NO: 30; or
   e) SEQ ID NO: 31.
7. A DNA molecule comprising at least one sequence that, upon transcription, generates an RNA molecule comprising a hammerhead type I ribozyme in its 5' end and a hammerhead type III ribozyme in its 3' end.
8. A DNA molecule comprising at least one sequence that, upon transcription, generates an RNA molecule comprising the synthetic catalytic polyribonucleotide of any one of 1 to 3, or the synthetic self-catalytic polyribonucleotide of any of 4 to 6.
9. The DNA molecule according to 8, wherein the DNA molecule comprises the synthetic self-catalytic polyribonucleotide of any of 4 to 6 in its 5' end.
10. The DNA molecule according to 9, wherein the DNA molecule further comprises a sequence in the 3' end that, upon transcription, results in a hammerhead type III ribozyme, preferably Elvd.
11. A composition comprising the catalytic polyribonucleotide defined in any of 1 to 3, the synthetic self-catalytic polyribonucleotide of any of 4 to 6, or the DNA molecule as defined in any of 7 to 10.
12. The catalytic polyribonucleotide as defined in any of 1 to 3, the synthetic self-catalytic polyribonucleotide of any of 4 to 6, the DNA molecule as defined in any of 7 to 10, or the composition as defined in 11, for use in medicine.
13. An *in vivo* or *in vitro* method of specifically cleaving the substrate polyribonucleotide of SEQ ID NO: 2, using the synthetic catalytic polyribonucleotide defined in any of 1 to 3, or the synthetic self-catalytic polyribonucleotide of any of 4 to 6.
14. A method to provide a plurality of RNA molecules characterized by having a sequence in the 5' end that is identical or substantially identical in all of the molecules, the method comprising the steps of:
   a) Providing a plurality of DNA molecules characterized in that, when transcribed, result in a plurality of RNA molecules, each of said RNA molecules being characterized by comprising the synthetic self-catalytic polyribonucleotide as defined in any one of 4 to 6 located in the 5' end, and
   b) *In vitro* or *in vivo* transcribing the plurality DNA molecules of a), thereby producing a plurality of RNA molecules that are self-cleaved by action of the synthetic self-catalytic polyribonucleotide.
15. A method to produce a circular RNA molecule, the method comprising the steps of:
   a) Providing a DNA molecule characterized in that, when transcribed, results in an RNA molecule comprising a first and a second regions, wherein:
      i. the first region comprises the synthetic self-catalytic polyribonucleotide as defined in any one of 4 to 6, and
      ii. the second region comprises:
         1. a self-catalytic polyribonucleotide that, when cleaved, generates an end capable of being ligated to the end generated after the self-cleavage of the synthetic self-catalytic polyribonucleotide of the first region, or
         2. a sequence capable of being ligated to the end generated after the self-cleavage of the synthetic self-catalytic polyribonucleotide of the first region,
   b) *in vitro* or *in vivo* transcribing the DNA molecule of step a), thereby producing an RNA molecule that is self-cleaved by action of the synthetic self-catalytic polyribonucleotide of the first region and of the self-catalytic polyribonucleotide of the second region, if present, so that a RNA molecule with ligatable ends is obtained, and
   c) adding a ligase capable of joining the 5' and 3' end of the cleaved RNA molecule obtained in step b), to provide a circular RNA molecule.
16. The method according to 15, wherein the first region is located in the 5' end of the RNA molecule, and the second region is located in the 3' end of the RNA molecule.
17. A circular RNA molecule obtained or obtainable from the method defined in 15 or 16.
18. A method of obtaining a circular RNA molecule, the method comprising the steps of:
   a) providing a DNA molecule characterized in that, when transcribed, results in an RNA molecule comprising a hammerhead type I ribozyme in its 5' end and a hammerhead type III ribozyme in its 3' end,
   b) in vitro or in vivo transcribing the DNA molecule of step a), thereby producing an RNA molecule that is self-cleaved by action of the hammerhead type I and II ribozymes, so that a RNA molecule with ligatable ends is obtained, and
   c) adding a ligase capable of joining the 5' and 3' end of the cleaved RNA molecule obtained in step b), to provide a circular RNA molecule.
19. The method according to 18, wherein the hammerhead type I ribozyme is the synthetic self-catalytic polyribonucleotide as defined in any one of 4 to 6.
20. The method according to 19, wherein the hammerhead type I ribozyme is selected from the group of SEQ ID NO: 21, 22, 29-31.
21. The method according to any one of 18-20, wherein the hammerhead type III ribozyme is Elvd ribozyme, preferably of SEQ ID NO: 32.
22. The method according to any one of 18-21, wherein the ligase of step c) is RtcB Ligase.

### GENERAL DEFINITIONS

It must be noted that, as used herein, the singular forms "a", "an", and "the", include plural references unless the context clearly indicates otherwise. Further, unless otherwise indicated, the term "at least" preceding a series of elements is to be understood to refer to every element in the series. Those skilled in the art will recognize or be able to ascertain using no more than routine experimentation, many equivalents to the specific embodiments of the invention described herein. Such equivalents are intended to be encompassed by the present invention.

If the term "about" as used in connection with a numerical value throughout the specification and the claims denotes an interval of accuracy, familiar and acceptable to a person skilled in the art. For instance, the term "about" means the indicated value ± 1% of its value, or the term "about" means the indicated value ± 2% of its value, or the term "about" means the indicated value ± 5% of its value, the term "about" means the indicated value ± 10% of its value, or the term "about" means the indicated value ± 20% of its value, or the term "about" means the indicated value ± 30% of its value; preferably the term "about" means exactly the indicated value (± 0%).

As used herein, the conjunctive term "and/or" between multiple recited elements is understood as encompassing both individual and combined options. For instance, where two elements are conjoined by "and/or", a first option refers to the applicability of the first element without the second. A second option refers to the applicability of the second element without the first. A third option refers to the applicability of the first and second elements together. Any one of these options is understood to fall within the meaning, and therefore satisfy the requirement of the term "and/or" as used herein. Concurrent applicability of more than one of the options is also understood to fall within the meaning, and therefore satisfy the requirement of the term "and/or."

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step. When used herein the term "comprising" can be substituted with the term "containing" or "including" or sometimes when used herein with the term "having". Any of the aforementioned terms (comprising, containing, including, having), whenever used herein in the context of an aspect or embodiment of the present invention may be substituted with the term "consisting of", though less preferred.

When used herein "consisting of" excludes any element, step, or ingredient not specified in the claim element. When used herein, "consisting essentially of does not exclude materials or steps that do not materially affect the basic and novel characteristics of the claim.

By "plurality" is referred herein as two or more nucleic acid molecules, including DNA, RNA, and DNA/RNA molecules.

The term "polynucleotide" refers to a polymer of at least 8 nucleotides in length selected from ribonucleotides, deoxyribonucleotides, or modified forms thereof. If the polynucleotide comprises ribonucleotides, it is referred to as "polyribonucleotide". When a polyribonucleotide has the ability of carrying out enzymatic catalysis or cleavage over a substrate polyribonucleotide, it is called herein a "catalytic polyribonucleotide".

Accordingly, the terms "catalytic polyribonucleotide" or refers to an RNA sequence (RNA enzyme) capable of cleaving a substrate polyribonucleotide when it hybridizes to it. As used herein "capable of cleaving" or "capable of exerting a catalytic cleavage activity" refers to an enzymatic reaction carried out by a catalytic polyribonucleotide over a substrate polyribonucleotide, that results in the cleavage of said substrate polyribonucleotide. The term "substrate polyribonucleotide" refers to a polyribonucleotide molecule that is cleaved by the action of the catalytic polyribonucleotide.

As used herein, a "self-catalytic polyribonucleotide" or "self-cleaving polyribonucleotide" refers to a single polyribonucleotide molecule having a RNA sequence comprising at least two regions, wherein a first region comprises a catalytic polyribonucleotide as defined above, and the second region comprises a substrate polyribonucleotide, as also defined above, and that is cleaved by the catalytic polyribonucleotide. The first and second regions comprised in the molecule may be connected by a linker, preferably a linker polyribonucleotide sequence. The self-catalytic or self-cleaving polyribonucleotide is also called herein "self-catalytic ribozyme" or "self-cleaving ribozyme". The terms "self-catalytic" and "self-cleaving" are considered synonymous herein.

Therefore, two RNA molecules or polyribonucleotides are defined herein: those called herein "catalytic polyribonucleotide" that comprise the sequence of the catalytic polyribonucleotide but do not comprise the sequence of the substrate polyribonucleotide, and those molecules called herein "self-catalytic polyribonucleotide", "self-cleaving polyribonucleotide", "self-cleaving ribozyme" or "self-catalytic ribozyme" that have both sequences, namely the catalytic polyribonucleotide and the substrate polyribonucleotide.

As used herein, the term nucleotides or nucleotide analogues is not limited in particular, and is meant to include deoxynucleoside triphosphates (dNTPs) such as for example, but not limited to dATP, dCTP, dGTP, dTTp, dITP, dUTP or derivatives thereof. As non limiting examples of such derivatives reference is made to dideoxynucleotides such as ddATP, ddCTP, ddGTP, ddTTp, ddITP, ddUTP, or oxidized derivatives like 8oxoA, 8oxoG, 50HC, 5OHU, or labelled derivatives like fluorescent labelled derivatives or even more complex labels like the labelled used for third generation sequencing.

### DESCRIPTION OF THE EMBODIMENTS

There is a need to develop more efficient ribozymes that show improved activity and function in mammalian cells with high efficiency. Example 1 shows the *in vitro* cleavage efficiency of 7 constructs, each with a different synthetic self-cleaving ribozyme in the 5' region. A comparison is made between 4 previously known naturally occurring hammerhead ribozymes (from *Ricordea florida, Trichobilharzia regent* and *Schistosoma rodhaini* -2 sequences-) and three new artificial designed ribozymes. As shown in Figure 1, the three artificial self-cleaving ribozymes showed better self-cleavage than the natural occurring ones, resulting in a RNA fragment of approximately 300 nt.

Example 2 shows the *in vitro* cleavage efficiency of 7 precursor circRNAs each flanked by one of the self-cleaving ribozymes analyzed in example 1. As shown in Figure 2, the three precursor circRNA containing the three artificial self-cleaving ribozymes showed a double self-cleavage resulting in a circRNA fragment of approximately 300 nt.

Lastly, Example 3 shows the circularization efficiency of 7 precursor circRNAs from Example 2, each flanked by one of the 5' HH ribozymes analyzed in example 1 and the HH ELVd ribozyme in the 3' region. As shown in Figure 3, the three precursor circRNA containing the three artificial self-cleaving ribozymes showed a 100% circularization efficiency, which is a higher efficiency than the natural occurring ribozymes.

In view of these results, the present invention provides polynucleotides that are capable of cleaving substrate polynucleotides. Preferably, the polynucleotides provided herein are catalytic polyribonucleotides, and the substrate polynucleotides are substrate polyribonucleotides. Preferably, the catalytic polyribonucleotides provided herein are synthetic. "Synthetic catalytic polyribonucleotide" means a catalytic polyribonucleotide which is not a naturally-occurring RNA catalyst, although they can be truncated or altered versions of naturally-occurring ribozymes. The terms "synthetic catalytic polyribonucleotide" also includes catalysts synthesized *in vitro* and catalysts synthesized *in vivo.* Thus, in the context of the present invention, "synthetic" is synonymous to "artificial", i.e., they are human made catalytic polyribonucleotides or ribozymes.

Particularly, three artificial catalytic polyribonucleotides were designed in the present invention. The sequences of them correspond to SEQ ID NOs: 23, 25 and 27, wherein their substrate polyribonucleotide correspond to SEQ ID NO: 24, 26 and 28, respectively. However, the polyribonucleotide sequences of said catalytic polyribonucleotides can be grouped and represented by the general formula as defined in SEQ ID NO: 33, wherein the substrate polyribonucleotide corresponds to the general formula of SEQ ID NO: 20.

In view of this, **a first aspect** of the present invention provides a synthetic catalytic polyribonucleotide, also referred as the **"catalytic polyribonucleotide of the invention",** comprising or consisting of SEQ ID NO: 33, wherein N₁, N₂, can be any nucleotide provided that N₁ in SEQ ID NO: 33 is capable of base pair with N₄ in SEQ ID NO: 20, and provided that N₂ in SEQ ID NO: 33 is capable of base pair with N₃ in SEQ ID NO: 20. In an embodiment, said synthetic catalytic polyribonucleotide of SEQ ID NO: 33 is capable of exerting a catalytic cleavage activity on a substrate polyribonucleotide having the sequence of SEQ ID NO: 20, wherein N₃, N₄, can be any nucleotide, provided that N₄ in SEQ ID NO: 20 is capable of base pair with N₁ in SEQ ID NO: 33, and provided that N₃ in SEQ ID NO: 20 is capable of base pair with N₂ in SEQ ID NO: 33. Therefore, in both SEQ ID NO: 33 and 20, the nucleotides N₁, N₂, N₃, and N₄ can be any nucleotides, provided that N₁ is capable of base pair with N₄ and N₂ is capable of base pair with N₃. A "base pair" refers to a specific combination of two nucleotides of nucleic acids connected via hydrogen bonds. A combination of nucleotides capable of forming a base pair are said to be "complementary" to each other. In DNA, adenine (A) pairs with thymine (T) and guanine (G) pairs with cytosine (C), while in RNA, A pairs with uracil(U) and G pairs with C. In RNA, so-called non-Watson-Crick base pairs such as G-A, G-U also occur as thermodynamically stable base pairs, and these combinations are also said to be "complementary" herein.

In an embodiment, the synthetic catalytic polyribonucleotide of the invention comprises or consists of SEQ ID NO: 23. In a preferred embodiment, the synthetic catalytic polyribonucleotide of the invention comprises or consists of SEQ ID NO: 23, wherein said synthetic catalytic polyribonucleotide is capable of exerting a catalytic cleavage activity on a substrate polyribonucleotide having the base sequence of SEQ ID NO: 24.

In another embodiment, the synthetic catalytic polyribonucleotide of the invention comprises or consists of SEQ ID NO: 25. In a preferred embodiment, the synthetic catalytic polyribonucleotide of the invention comprises or consists of SEQ ID NO: 25, wherein said synthetic catalytic polyribonucleotide is capable of exerting a catalytic cleavage activity on a substrate polyribonucleotide having the base sequence of SEQ ID NO: 26.

In another embodiment, the synthetic catalytic polyribonucleotide of the invention comprises or consists of SEQ ID NO: 27. In a preferred embodiment, the synthetic catalytic polyribonucleotide of the invention comprises or consists of SEQ ID NO: 27, wherein said synthetic catalytic polyribonucleotide is capable of exerting a catalytic cleavage activity on a substrate polyribonucleotide having the base sequence of SEQ ID NO: 28.

Thus, the catalytic polyribonucleotides according to the invention are synthetic or artificial catalytic RNA sequences that are capable of recognizing and cleaving a substrate polyribonucleotide. The catalytic cleavage may occur in *cis* or *trans,* depending on whether the substrate polyribonucleotide is part of the same molecule as the catalytic polyribonucleotide (cis cleavage) or is part of a different molecule *(trans* cleavage). As used herein and in the technical field of the present invention, a cleavage "in *cis"* means a cleavage happening locally within the same molecule (i.e., intramolecular cleavage). A cleavage "in *trans*" means that the cleavage is happening in a different molecule (i.e., intermolecular cleavage).

Thus, in the case of the *trans* cleavage or intermolecular cleavage, the synthetic catalytic polyribonucleotides of the invention are capable of cleaving a substrate polyribonucleotide comprised in a nucleic acid molecule that is different from the molecule where said catalytic polyribonucleotide of the invention is comprised. In an embodiment, the substrate polyribonucleotide is comprised in a nucleic acid of interest, such as the transcriptome of an organism. Said organism can be a virus, a bacteria, a fungi, or a eukaryotic cell. Preferably, said transcriptome is the RNA or mRNA molecules expressed by said organism. Preferably, the substrate polyribonucleotide in the case of a *trans* cleavage is a polyribonucleotide sequence, preferably a mRNA sequence, comprised in the transcriptome of a human pathogen.

In the case of the *cis* cleavage or intramolecular cleavage, the synthetic catalytic polyribonucleotide and its corresponding substrate polyribonucleotide are comprised in the same molecule, thereby forming a molecule that has a self-cleavage ability, named herein a self-catalytic polyribonucleotide or self-catalytic ribozyme. Said self-catalytic polyribonucleotide/ribozyme is characterized by comprising a catalytic polyribonucleotide and its corresponding substrate polyribonucleotide.

In view of this, the present invention also provides in the first aspect self-catalytic polyribonucleotides comprising at least two regions, wherein a first region comprises the catalytic polyribonucleotide of the invention (defined above), and the second region comprises its corresponding substrate polyribonucleotide. This molecule is also referred herein as the **"self-catalytic polyribonucleotide of the invention",** or **"self-catalytic ribozyme of the invention".**

Similar as for the catalytic polyribonucleotide of the invention, the self-catalytic polyribonucleotides of the invention are synthetic, i.e., non-natural molecules. The term **"polyribonucleotide of the invention"** is used from herein after to refer to both the catalytic polyribonucleotides of the invention and the self-catalytic polyribonucleotides/ribozymes of the invention.

In an embodiment, the catalytic polyribonucleotide and the substrate polyribonucleotide comprised in the synthetic self-catalytic polyribonucleotide of the invention are connected by means of a linker. Preferably, the linker is a nucleotide sequence, preferably GAUA.

SEQ ID NOs: 29-31 represent examples of the synthetic self-catalytic polyribonucleotides of the invention where the catalytic polyribonucleotide and its corresponding substrate polyribonucleotide are comprised in the same molecule, thereby exerting a *cis* cleavage. Said synthetic self-catalytic polyribonucleotides can be grouped and represented by the general sequence of SEQ ID NOs: 21 or 22. Thus, also part of the invention is a synthetic self-catalytic polyribonucleotide that comprises or consists of SEQ ID NO: 21, wherein N₁, N₂, N₃, and N₄ can be any nucleotides, provided that N₁ is capable of base pair with N₄ and N₂ is capable of base pair with N₃, and wherein N₅ is a linker, preferably the nucleotide sequence "GAUA". In another embodiment, the synthetic self-catalytic polyribonucleotide comprises or consists of SEQ ID NO: 22, wherein N₁, N₂, N₃, and N₄ can be any nucleotides, provided that N₁ is capable of base pair with N₄ and N₂ is capable of base pair with N₃. In an embodiment, the synthetic self-catalytic polyribonucleotide comprises or consists of SEQ ID NO: 29, 30, or 31. These self-catalytic polyribonucleotides have a structure compatible with type I hammerhead-like ribozymes. A hammerhead ribozyme (HHR) is a small (50-150 nucleotides) catalytic RNA motif capable of endonucleolytic (self-) cleavage. It is composed of a catalytic core of conserved nucleotides flanked by three helices (helices I, II and III). Although HHRs that cleave other RNAs in trans have been identified, the majority of known natural HHRs are cis-, i.e., self-cleaving entities that contain the motif within a single RNA strand. This gives rise to the three circularly permuted forms that are named types I, II, or III, according to the open-ended helix that connects the motif with the flanking sequences.

In an embodiment, the synthetic catalytic polyribonucleotide of the invention is comprised in or is a portion of a larger RNA molecule. As used herein, the term "portion" or "region" when referred to a nucleotide sequence means a nucleotide region or fragment of said sequence. This is for example the case of the self-catalytic polyribonucleotides described above, which are RNA molecules where a portion of said molecule comprises the catalytic polyribonucleotide of the invention and another portion comprises its substrate polyribonucleotide. An RNA molecule comprising at least one of the polyribonucleotides of the invention is referred herein as the **"RNA molecule of the invention".** When the RNA molecule of the invention comprises at least one self-catalytic polyribonucleotide of the invention is called **"self-catalytic RNA molecule of the invention"** or **"self-cleaving RNA molecule of the invention".**

Preferably, the RNA molecule of the invention comprises one or more of the polyribonucleotide of the invention in its 5' and/or 3' region. More preferably, the RNA molecule of the invention comprises a self-catalytic polyribonucleotide of the invention in its 5' and/or 3' end. The expression "3' region", as used herein, refers to a region of a nucleotide strand that includes the 3' end of said strand. As used herein, the "3' region" and the "3' terminal region" refer to the same region of a nucleotide strand and are used interchangeably. The term "3' end", as used herein, designates the end of a nucleotide strand that has the hydroxyl group of the third carbon in the sugar-ring of the ribose or deoxyribose at its terminus. The expression "5' region", as used herein, refers to a region of a nucleotide strand that includes the 5' end of said strand. As used herein, the "5' region" and the "5' terminal region" refers to the same region of a nucleotide strand and may be used interchangeably. The term "5' end", as used herein, designates the end of a nucleotide strand that has the fifth carbon in the sugar-ring of the deoxyribose at its terminus. Most preferably, the self-polyribonucleotide of the invention is placed in the 5' end of the RNA molecule of the invention where it is comprised.

A nucleic acid molecule may comprise or encode for one or more of the polyribonucleotides, including the catalytic polyribonucleotides and/or the self-catalytic polyribonucleotides of the invention. In an embodiment, a nucleic acid molecule may also comprise or encode for other self-catalytic ribozymes or other self-catalytic polyribonucleotides, so that they are used in combination. In an embodiment, said other self-catalytic ribozymes or other self-catalytic polyribonucleotide that are used in combination with the polyribonucleotides of the invention are hammerhead ribozymes, preferably hammerhead type III ribozyme, most preferably Elvd.

Preferably, the RNA molecule of the invention comprises two self-catalytic polyribonucleotides of the invention, one in each end of the molecule. Preferably, the RNA molecule of the invention comprises a first and a second self-catalytic polyribonucleotides, wherein the first self-catalytic polyribonucleotide is a self-catalytic polyribonucleotide of the invention, and the second self-catalytic polyribonucleotide is a known self-catalytic polyribonucleotide, such as a hammerhead ribozyme, preferably hammerhead type III ribozyme, most preferably Elvd. The term "known" as used herein refers to a self-catalytic ribozyme that either "natural", namely found in nature, or "synthetic", i.e., previously sequenced and characterized.

When the RNA molecule of the invention comprises two self-catalytic polyribonucleotides, it will be self-cleaved twice, resulting in three RNA molecules: a first RNA molecule corresponding to the 5' region of the original RNA molecule of the invention, a second RNA molecule corresponding to the region of the original RNA molecule that was flanked by the two self-catalytic polyribonucleotides, and a third RNA molecule corresponding to the 3' region of the original RNA molecule of the invention. The second RNA molecule may be subsequently circularized (i.e., by ligation using a suitable ligase), thereby forming a circular RNA (also called herein circRNA).

Preferably, the RNA molecule of the invention comprises, before cleavage, a first and a second self-catalytic polyribonucleotides, wherein:
- the first self-catalytic polyribonucleotide is a self-catalytic polyribonucleotide of the invention, and
- the second self-catalytic polyribonucleotide is a self-catalytic polyribonucleotide, such as a hammerhead ribozyme, preferably hammerhead type III ribozyme, most preferably Elvd;
where the first self-catalytic polyribonucleotide is placed in the 5' region, preferably 5' end, of the RNA molecule, and wherein the second self-catalytic polyribonucleotide is placed in the 3' region, preferably 3' end, of the RNA molecule. As explained above, this RNA molecule is self-cleaved due to the presence of the two self-catalytic polyribonucleotides, and one of the resulting cleaved RNA molecule (called the second RNA molecule above) may be circularized to form a circRNA. The circular RNA molecule generated after the self-cleavage of an original RNA molecule of the invention and the subsequent ligation of the 5' and 3' ends of the second RNA molecule resulting from the cleavage is called herein the "circRNA of the invention".

By "ELVd ribozyme" is referred herein to the Eggplant latent viroid self-catalytic ribozyme or polyribonucleotide that consists of a single-stranded, circular, non-coding RNA of 332-335 nucleotides that folds in a branched quasi-rod-like minimum free-energy conformation. Preferably, said ELVd self-catalytic polyribonucleotide/ribozyme comprises or consists of SEQ ID NO: 32.

In an embodiment, the present invention provides a self-cleaving RNA molecule of the invention that comprises, before cleavage, a first and a second self-catalytic polyribonucleotides or ribozymes, wherein:
- the first self-catalytic polyribonucleotide is a self-catalytic polyribonucleotide of the invention, preferably selected from the list consisting of SEQ ID NOs: 21, 22, or 29-31; and
- the second self-catalytic polyribonucleotide is a self-catalytic polyribonucleotide, preferably Elvd;
where the first self-catalytic polyribonucleotide is placed in the 5' end of the RNA molecule, and wherein the second self-catalytic polyribonucleotide is placed in the 3' end of the RNA molecule.

It is important to note that the self-cleaving RNA molecule of the invention that is the precursor of the circRNA of the invention does not necessarily need to have two self-catalytic polyribonucleotides. It is sufficient if said RNA molecule has only one self-catalytic polyribonucleotide, preferably a self-catalytic polyribonucleotide of the invention, provided that the RNA molecule comprises a compatible end capable of being ligated to the ends that said self-catalytic polyribonucleotide creates after its cleavage activity. As used herein, the term "compatible ends capable of being ligated" refers to RNA overhangs that can be joined by ligation, i.e., that are ligatable ends.

Hence, in an embodiment, the present invention provides a self-cleaving RNA molecule of the invention that comprises, before cleavage, a first and a second regions, wherein:
- the first region comprises a self-catalytic polyribonucleotide of the invention, preferably selected from the list consisting of SEQ ID NOs: 21, 22, or 29-31; and
- the second region comprises:
   1. a self-catalytic polyribonucleotide that, when cleaved, generates a compatible end capable of being ligated to the end generated after the self-cleavage of the self-catalytic polyribonucleotide comprised in the first region, or
   2. a sequence capable of being ligated to the end generated after the self-cleavage of the self-catalytic polyribonucleotide comprised in the first region;
where the first region is placed in the 5' end of the RNA molecule, and wherein the second region is placed in the 3' end of the RNA molecule.

The present invention also provides a circular RNA molecule of the invention obtained or obtainable by the method comprising the steps of:
a) providing a self-cleaving RNA molecule comprising, before cleavage, a first and a second regions wherein:
   i. the first region comprises a self-catalytic polyribonucleotide of the invention, and
   ii. the second region comprises:
      1. a self-catalytic polyribonucleotide that, when cleaved, generates a compatible end capable of being ligated to the end generated after the self-cleavage of the self-catalytic polyribonucleotide of the first region, or
      2. a sequence capable of being ligated to the end generated after the self-cleavage of the self-catalytic polyribonucleotide of the first region,
      wherein, preferably, the first region is placed in the 5' end of the RNA molecule, and the second region is placed in the 3' end of the RNA molecule, and
b) allowing the RNA molecule to be cleaved by action of the self-catalytic polyribonucleotide of the first region and, optionally, of the self-catalytic polyribonucleotide of the second region, and
c) ligating the ends of the cleaved RNA molecule resulting from step b), thereby obtaining the circular RNA molecule of the invention.

In the context of the present invention, "a sequence capable of being ligated to the end generated after the self-cleavage of the self-catalytic polyribonucleotide of the first region" refers to a sequence that has a compatible end with the end generated after the self-cleavage of the self-catalytic polyribonucleotide comprised in the first region. This sequence can be synthetised *in vitro* or may be generated by enzymatic reaction.

The expression "allowing the RNA molecule to be cleaved" is interpreted herein as to providing the suitable reaction conditions to promote that the self-catalytic polyribonucleotide comprised in the RNA molecule is automatically activated and carries out the self-cleavage. Said conditions are known in the art and are also exemplified in Examples 1-3 below.

In an embodiment, the circular RNA molecule of the invention obtained or obtainable by the method comprising the steps of:
a) providing a self-cleaving RNA molecule comprising, before cleavage, a first and a second regions wherein:
   i. the first region comprises a self-catalytic polyribonucleotide of the invention, and
   ii. the second region comprises:
      1. a self-catalytic polyribonucleotide that is a hammerhead type III ribozyme, most preferably Elvd, or
      2. a sequence capable of being ligated to the end generated after the self-cleavage of the self-catalytic polyribonucleotide of the first region,
      wherein the first region is placed in the 5' end of the RNA molecule, and the second region is placed in the 3' end of the RNA molecule, and
b) allowing the RNA molecule to be cleaved by action of the self-catalytic polyribonucleotide of the first region and, optionally, of the self-catalytic polyribonucleotide of the second region, and
c) ligating the ends of the cleaved RNA molecule resulting from step b), thereby obtaining the circular RNA molecule of the invention.

In an embodiment, the circular RNA molecule of the invention obtained or obtainable by the method comprising the steps of:
a) providing a self-cleaving RNA molecule comprising, before cleavage, a first and a second regions wherein:
   i. the first region comprises a self-catalytic polyribonucleotide selected from the group consisting of SEQ ID NOs: 21, 22, 29, 30 and 31, and
   ii. the second region comprises a hammerhead type III ribozyme, preferably Elvd;
   wherein the first region is placed in the 5' end of the RNA molecule, and the second region is placed in the 3' end of the RNA molecule, and
b) allowing the RNA molecule to be cleaved by action of the self-catalytic polyribonucleotide of the first and the second regions, and
c) ligating the ends of the cleaved RNA molecule resulting from step b), thereby obtaining the circular RNA molecule of the invention, wherein the ligase is preferably the RtcB Ligase.

By "RtcB ligase" is referred herein to as a ligase from *E. coli* that joins single stranded RNA with a 3'-phosphate or 2',3'-cyclic phosphate to another RNA with a 5' -hydroxyl.

Therefore, the following self-cleaving RNA molecules are encompassed by the present invention:
(i) a self-cleaving RNA molecule of the invention comprising in its 5' end a self-catalytic polyribonucleotide of the invention and its 3' end the self-catalytic polyribonucleotide ELVd;
(ii) a self-cleaving RNA molecule of the invention comprising in its 5' end the self-catalytic polyribonucleotide of SEQ ID NO: 21 and its 3' end the self-catalytic polyribonucleotide ELVd;
(iii) a self-cleaving RNA molecule of the invention comprising in its 5' end the self-catalytic polyribonucleotide of SEQ ID NO: 22 and its 3' end the self-catalytic polyribonucleotide ELVd;
(iv) a self-cleaving RNA molecule of the invention comprising in its 5' end the self-catalytic polyribonucleotide of SEQ ID NO: 29 and its 3' end the self-catalytic polyribonucleotide ELVd;
(v) a self-cleaving RNA molecule of the invention comprising in its 5' end the self-catalytic polyribonucleotide of SEQ ID NO: 30 and its 3' end the self-catalytic polyribonucleotide ELVd; or
(vi) a self-cleaving RNA molecule of the invention comprising in its 5' end the self-catalytic polyribonucleotide of SEQ ID NO: 31 and its 3' end the self-catalytic polyribonucleotide ELVd.

It is noted that the self-cleaving RNA molecules as defined in (i) to (vi) can be circularized using a suitable ligase, such as the RtcB ligase, thereby forming the circRNA of the invention.

Those skilled in the art can prepare the polyribonucleotides (including the catalytic polyribonucleotides and the self-catalytic polyribonucleotides) of the invention by synthesizing a RNA nucleic acid molecule having one or more polyribonucleotides explained above. The synthesis of the polyribonucleotide can be performed by any method commonly used by those skilled in the art. For example, the self-catalytic polyribonucleotides of the invention can be produced in the form of a DNA molecule that encodes or that, upon transcription, produces, the polyribonucleotide of the invention, amplify it by PCR to prepare a template DNA and transcribe it by T7RNA polymerase to synthesize the intended polyribonucleotide. This is shown in Example 1, where seven DNA templates encoding for the ribozymes were generated. Thus, encompassed by the present invention are also molecules (DNA, RNA, and DNA/RNA hybrids) that encode for or result in the molecules (i) to (vi) described above. For instance, in an embodiment, a DNA molecule that encodes the molecules (i) to (vi) may be the DNA molecules (also called circRNA precursors) of SEQ ID NOS: 16, 17 and 18.

Thus, a DNA molecule encoding for one or more of the polyribonucleotides defined above (including the catalytic polyribonucleotides, the self-catalytic polyribonucleotides, the RNA molecules, and the cirRNA molecules of the invention) is also included in the present invention. This DNA molecule is also referred herein as the "DNA molecule of the invention". Said DNA molecule of the invention produces, upon transcription, the polyribonucleotides of the invention. In an embodiment, the DNA molecule of the invention comprises or consists of SEQ ID NOs: 5, 6, or 7.

It is important to note that, in the methods or steps described in the present invention that involve the generation of a RNA molecule, for instance the RNA molecule or the circRNA molecule of the invention, departing from a DNA molecule, a step of *in vitro* or *in vivo* transcription is needed. This is to generate the RNA molecule that will automatically be self-cleaved if the polyribonucleotides of the invention are comprised in said RNA molecule. The DNA molecule should thus be designed to include the necessary elements for said step of *in vitro* or *in vivo* transcription. A skilled in the art knows how to perform transcriptions. For example, the DNA molecule may comprise transcription initiation sites or a suitable promoter. Preferably, the DNA molecule that is a precursor of the RNA of the invention comprises a suitable promoter such as T7 promoter.

In an embodiment, the DNA molecule encodes at least two self-cleaving ribozyme, wherein a first self-cleaving ribozyme is a self-catalytic polyribonucleotide of the invention, and a second self-cleaving ribozyme is a known (i.e., natural or synthetic) hammerhead ribozyme, preferably hammerhead type III ribozyme, most preferably Elvd, and wherein the sequence encoding the first self-catalytic polyribonucleotide of the invention is placed in the 5' region, preferably 5' end, of said DNA molecule, and the sequence encoding the second self-cleaving ribozyme is placed in the 3' region, preferably 3' end, of said DNA molecule.

In an embodiment, DNA molecule of the invention encodes or upon transcription results in the circRNA of the invention. This is exemplified in Examples 2 and 3, where seven DNA molecules were generated, of which three of them (SEQ ID NOs: 16, 17, and 18) are precursors molecule that, upon transcription, generate a RNA of the invention, that is subsequently cleaved and ligated, thereby resulting in the circular RNA of the invention. Therefore, provided herein are DNA molecules of the invention comprising or consisting of SEQ ID NO: 16, 17 or 18.

In view of all of the above embodiments, it is then clear that also part of the present invention are:
(a) an RNA molecule comprising one or more of the polyribonucleotides of the invention;
(b) an RNA consisting of a sequence complementary to the RNA of (a) above;
(c) a DNA consisting of a sequence identical to the RNA of (a) above, but U is replaced by T;
(d) a DNA consisting of a sequence identical to the RNA of (b) above, but U is replaced by T.

Preferably, the molecules (a) to (d) defined herein comprise or encode for any of SEQ ID NOs: 33, 21, 22, 23, 25, 27, 29, 30 or 31. The molecules (a) to (d) defined above may further comprise or encode for other ribozymes, such as hammerhead ribozymes, preferably hammerhead type III ribozyme, most preferably Elvd.

Included in the first aspect of the invention are compositions comprising one or more of the molecules defined herein, including the polyribonucleotides (catalytic polyribonucleotides and self-catalytic polyribonucleotides) of the invention, and DNA and RNA molecules encoding or comprising, respectively, thereof.

Included in the first aspect of the invention are also kits comprising one or more of the molecules defined herein, including the polyribonucleotides (catalytic polyribonucleotides and self-catalytic polyribonucleotides) of the invention, and DNA and RNA molecules encoding or comprising, respectively, thereof.

Lastly, it is noted that the polyribonucleotides of the invention may be provided in the form of a system of two or more molecules that are capable of interacting with each other and give rise to the polyribonucleotides of the invention.

A **second aspect** of the present invention refers to the use of the molecules and compositions as defined in the first aspect, in medicine or for the generation of a medicament. Especially, the polyribonucleotides (catalytic polyribonucleotides and self-catalytic polyribonucleotides) of the invention or compositions comprising thereof may be used in the therapeutic field as molecules capable of targeting sequences of interest, that are substrate polyribonucleotides. Preferably, the synthetic catalytic polyribonucleotides are used in the medical field or in a method of treatment by therapy. For example, a catalytic polyribonucleotide of the invention may be used to produce the *trans* cleavage of the transcriptome of a virus, if the transcriptome of said virus comprises its corresponding substrate polyribonucleotide, thereby inhibiting the viral replication. Therefore, the polyribonucleotides of the invention, particularly the catalytic polyribonucleotides of the invention, can be used in medicine.

Catalytic ribozymes show great therapeutic promise for altering viral replication *in vivo.* A therapeutic ribozyme is exposed to the substrate polynucleotide in one of two general ways. First, the catalytic ribozyme may be isolated in the laboratory and packaged in a suitable delivery vehicle. Second, the DNA encoding the ribozyme of interest is incorporated into a vector with a suitable promotor and delivered to the target cell.

A **third aspect** of the present invention refers to an *in vivo* or *in vitro* method of cleaving a substrate polyribonucleotide by using one or more of the polyribonucleotides of the invention. Preferably, the use refers to an *in vitro* or *in vivo* method for cleaving SEQ ID NO: 20, by using the catalytic polyribonucleotide of SEQ ID NO: 33, wherein N₁, N₂, N₃, and N₄ can be any nucleotides, provided that N₁ is capable of base pair with N₄ and N₂ is capable to base pair with N₃. Also included in the third aspect is the cleaved substrate polyribonucleotide obtained or obtainable from the method defined in said aspect.

A **fourth aspect** of the present invention refers to a method to produce the circular RNA molecule of the invention, the method comprising the steps of:
a) providing a DNA molecule characterized in that, when transcribed, results in an RNA molecule comprising at least a first and a second regions, wherein:
   i. the first region comprises a self-catalytic polyribonucleotide, preferably selected from the list consisting of SEQ ID NO: 21, 22 and 29-31, and
   ii. the second region comprises:
      1. a self-catalytic polyribonucleotide that, when cleaved, generates a compatible end capable of being ligated to the end generated after the self-cleavage of the self-catalytic polyribonucleotide of the first region, or
      2. a sequence capable of being ligated to the end generated after the self-cleavage of the self-catalytic polyribonucleotide of the first region,
b) *in vitro* or *in vivo* transcribing the DNA molecule of step a), thereby producing the self-cleavage of the self-catalytic polyribonucleotide of the first region and of the self-catalytic polyribonucleotide of the second region, if present, so that a RNA molecule with ligatable ends is obtained, and
c) adding a ligase capable of joining the 5' and 3' end of the RNA molecule obtained in b), to provide a circular RNA molecule.

Preferably, the self-catalytic polyribonucleotide that is comprised in the second region is a known (i.e., natural or synthetic) self-catalytic polyribonucleotide, such as a hammerhead ribozyme, preferably hammerhead type III ribozyme, most preferably Elvd. Preferably, said ELVd comprises SEQ ID NO: 32.

Preferably, the first region of the RNA molecule generated in step a) is placed in the 5' region, preferably 5' end, of said molecule, and the second region is placed in the 3' region, preferably 3' end, of said molecule.

Preferably, the method to produce the circular RNA molecule of the invention comprises the steps of:
a) Providing a DNA molecule characterized in that, when transcribed, results in an RNA molecule comprising at least a first and a second regions, wherein:
   i. the first region comprises a self-catalytic polyribonucleotide selected from the list consisting of SEQ ID NO: 21, 22 and 29-31, and
   ii. the second region comprises:
      1. a self-catalytic polyribonucleotide that is a hammerhead type III ribozyme, most preferably Elvd, or
      2. a sequence capable of being ligated to the end generated after the self-cleavage of the self-catalytic polyribonucleotide of the first region,
b) *in vitro* or *in vivo* transcribing the DNA molecule of step a), thereby producing an RNA molecule that is self-cleaved by action of the self-catalytic polyribonucleotide of the first region and the self-catalytic polyribonucleotide of the second region, if present, so that a RNA molecule with ligatable ends is obtained , and
c) adding a ligase capable of joining the 5' and 3' end of the cleaved RNA molecule obtained in step b), to provide a circular RNA molecule, wherein the ligase is preferably a RtcB ligase.

Preferably, the method to produce the circular RNA molecule of the invention comprises the comprising the steps of:
a) Providing a DNA molecule characterized in that, when transcribed, results in an RNA molecule comprising at least a first and a second regions, wherein:
   i. the first region comprises a self-catalytic polyribonucleotide selected from the list consisting of SEQ ID NO: 21, 22 and 29-31, and
   ii. the second region comprises a self-catalytic polyribonucleotide that is a hammerhead type III ribozyme, most preferably Elvd,
b) *in vitro* or *in vivo* transcribing the DNA molecule of step a), thereby producing an RNA molecule that is self-cleaved by action of the self-catalytic polyribonucleotides of the first and second regions, so that a RNA molecule with ligatable ends is obtained, and
c) adding a ligase capable of joining the 5' and 3' end of the cleaved RNA molecule obtained in step b), to provide a circular RNA molecule, wherein the ligase is preferably a RtcB ligase.

Step a) in the method of the fourth aspect comprises or consists of providing a DNA molecule. This DNA molecule is designed so that, when it is transcribed in step b), a RNA molecule is formed. As explained above, it is readily apparent for the skilled person that the design of such DNA molecule should take into account that said DNA molecule needs to comprise transcription initiation elements, such as suitable promoters, preferably a T7 promoter.

Step b) in the method of the fourth aspect comprises or consists of the *in vitro* or *in vivo* transcription of the DNA molecule provided in a), thereby producing an RNA molecule that is automatically self-cleaved by action of the self-catalytic polyribonucleotide of the first and second regions, if present, so that a RNA molecule with ligatable ends is obtained. A molecule with "ligatable ends" is a molecule whose 5' and 3' ends are compatible to be ligated by, e.g., action of a ligase. This step is common in the art and the skilled person knows how to provide the necessary reagents and conditions to promote the transcription of the DNA molecule provided in a). For example, a transcription step is shown in Example 2 of the present invention, where the DNA molecule of step a) comprises a T7 promoter, and it is thus in vitro transcribed using T7 RNA polymerase. The resultant RNAs are then self-cleaved, as shown in Fig. 2.

Lastly, step c) in the method of the fourth aspect comprises or consists of adding a ligase capable of joining the 5' and 3' end of the cleaved RNA molecule obtained in step b). The step of ligation the ends of an RNA molecule are known in the art. For example, a ligation step is shown in Example 3 of the present invention, where the linear RNAs obtained from Example 2 are circularized by incubating them under suitable conditions (i.e., 37°c for 1 hour) with RtcB ligase. Once the circular RNA of the invention are produced, a further optional step d) may be purifying them or removing the non-ligated RNA molecules by incubating the sample with an RNAse, such as an RNAse R.

Also included in the fourth aspect is the circular RNA molecule or molecules obtained or obtainable from the method defined in said aspect.

In a **fifth** aspect, the invention provides a method to provide a plurality of RNA molecules characterized by having a sequence in the 5' region that is identical or substantially identical in all of the molecules. A region is "substantially identical" to another region when the percentage of nucleobase identity between both regions is at least 95%, at least 96%, at least 97%, at least 98%, at least 99%, at least 99,9%, preferably at least 99,99%. Thus, the "substantial identity" includes the possible errors (i.e., insertion, deletion or substitution of nucleotides made by polymerase enzymes or by DNA damage, library processing, sequencing or mapping. The term "Identical" means a percentage of identity of 100%. In an embodiment, the "5' region" that is identical or substantially identical in the plurality of RNA molecule comprises about 60, 50, 40, 30, 25, 20 ,15, 10, 9, 8, 7, 6, 5, 4, 3, 2, 1 nucleotides from the 5' end of the RNA molecules.

In an embodiment, the sequence that is identical or substantially identical in the plurality of RNA molecules is located in the 5' end of each of said RNAs molecules.

Therefore, the plurality of RNA molecules obtained after the method of the fifth aspect all have the same sequence in their 5' region, preferably the same sequence in their 5' end. This is achieved following the method steps of:
a) Providing a plurality of DNA molecules characterized in that, when transcribed, result in a plurality of RNA molecules comprising a self-catalytic polyribonucleotide of the invention, and
b) *In vitro* or *in vivo* transcribing said a plurality of DNA molecules of step a), thereby producing a plurality of RNA molecules that are self-cleaved by action of the self-catalytic polyribonucleotide.

Preferably, the self-catalytic polyribonucleotide of the invention comprised in the RNA molecules produced in step a) is placed in the 5' region, preferably 5' end, of the each of the RNA molecules.

In an embodiment, the method according to the fifth aspect comprises the steps of:
a) Providing a plurality of DNA molecules characterized in that, when transcribed, result in a plurality of RNA molecules comprising a self-catalytic polyribonucleotide of the invention in their 5' end, and
b) *In vitro* or *in vivo* transcribing said a plurality of DNA molecules of step a), thereby producing a plurality of RNA molecules that are self-cleaved by action of the self-catalytic polyribonucleotide.

In an embodiment, the method according to the fifth aspect comprises the steps of:
a) Providing a plurality of DNA molecules characterized in that, when transcribed, result in a plurality of RNA molecules comprising a self-catalytic polyribonucleotide selected from the group consisting of SEQ ID NOs: 21, 22, 29, 30 and 31, and
b) *In vitro* or *in vivo* transcribing said a plurality of DNA molecules of step a), thereby producing a plurality of RNA molecules that are self-cleaved by action of the self-catalytic polyribonucleotide.

In an embodiment, the method of the fifth aspect may be performed by directly producing, i.e. by synthesis, the plurality of RNA molecules. In this case, the method would comprise the steps of:
a) Providing a plurality of RNA molecules comprising a self-catalytic polyribonucleotide of the invention, and
b) allowing the RNA molecule to be cleaved by action of the self-catalytic polyribonucleotide of the invention.

In an embodiment, the method according to the fifth aspect comprises the steps of:
a) Providing a plurality of RNA molecules comprising a self-catalytic polyribonucleotide selected from the group consisting of SEQ ID NOs: 21, 22, 29, 30 and 31, and
b) allowing the RNA molecule to be cleaved by action of the self-catalytic polyribonucleotide of the invention.

Also included in the fifth aspect is the plurality of RNA molecules obtained or obtainable from the method defined in said aspect.

In a **sixth aspect,** the present invention also provides a DNA molecule comprising least one sequence that, upon transcription, generates an RNA molecule comprising a hammerhead type I ribozyme in its 5' end and a hammerhead type III ribozyme in its 3' end. Preferably, the hammerhead type I ribozyme placed in the 5' end is the self-catalytic polyribonucleotide of the invention. Preferably, the hammerhead type III ribozyme placed in the 3' end is the Elvd ribozyme, preferably comprising or consisting of SEQ ID NO: 32. This DNA molecule can also be used as a precursor in a method to generate circular RNAs, since the RNA molecule comprising a hammerhead type I ribozyme in its 5' end and a hammerhead type III ribozyme in its 3' end will be self-cleaved, and a RNA molecule with ligatable ends will be obtained.

Therefore, the sixth aspect of the invention also refers to a method to generate circular RNAs, the method comprising the steps of:
a) Providing a DNA molecule characterized in that, when transcribed, results in an RNA molecule comprising a hammerhead type I ribozyme in its 5' end and a hammerhead type III ribozyme in its 3' end,
b) in vitro or in vivo transcribing the DNA molecule of step a), thereby producing an RNA molecule that is self-cleaved by action of the hammerhead type I and II ribozymes, so that a RNA molecule with ligatable ends is obtained, and
c) adding a ligase capable of joining the 5' and 3' end of the cleaved RNA molecule obtained in step b), to provide a circular RNA molecule.

Preferably, the sixth aspect of the invention also refers to a method to generate circular RNAs, the method comprising the steps of:
a) Providing a DNA molecule characterized in that, when transcribed, results in an RNA molecule comprising a hammerhead type I ribozyme in its 5' end and a hammerhead type III ribozyme in its 3' end, wherein the hammerhead type I ribozyme in the 5' end is one of the self-catalytic polyribonucleotides of the invention,
b) in vitro or in vivo transcribing the DNA molecule of step a), thereby producing an RNA molecule that is self-cleaved by action of the hammerhead type I and II ribozymes, so that a RNA molecule with ligatable ends is obtained, and
c) adding a ligase capable of joining the 5' and 3' end of the cleaved RNA molecule obtained in step b), to provide a circular RNA molecule.

Preferably, the sixth aspect of the invention also refers to a method to generate circular RNAs, the method comprising the steps of:
a) Providing a DNA molecule characterized in that, when transcribed, results in an RNA molecule comprising a hammerhead type I ribozyme in its 5' end and a hammerhead type III ribozyme in its 3' end, wherein the hammerhead type I ribozyme in the 5' end is selected from the group of SEQ ID NO: 21, 22, 29-31,
b) in vitro or in vivo transcribing the DNA molecule of step a), thereby producing an RNA molecule that is self-cleaved by action of the hammerhead type I and II ribozymes, so that a RNA molecule with ligatable ends is obtained, and
c) adding a ligase capable of joining the 5' and 3' end of the cleaved RNA molecule obtained in step b), to provide a circular RNA molecule.

Preferably, the sixth aspect of the invention also refers to a method to generate circular RNAs, the method comprising the steps of:
a) Providing a DNA molecule characterized in that, when transcribed, results in an RNA molecule comprising a hammerhead type I ribozyme in its 5' end and a hammerhead type III ribozyme in its 3' end, wherein the hammerhead type I ribozyme in the 5' end is selected from the group of SEQ ID NO: 21, 22, 29-31, and wherein the hammerhead type III ribozyme in the 3' end is Elvd ribozyme, prefrably the Elvd ribozyme of SEQ ID NO:32,
b) in vitro or in vivo transcribing the DNA molecule of step a), thereby producing an RNA molecule that is self-cleaved by action of the hammerhead type I and II ribozymes, so that a RNA molecule with ligatable ends is obtained, and
c) adding a ligase, preferably RtcB ligase, capable of joining the 5' and 3' end of the cleaved RNA molecule obtained in step b), to provide a circular RNA molecule.

Also included in the sixth aspect is the circular RNA molecule obtained or obtainable from the method defined in said aspect.

Each embodiment disclosed herein is contemplated as being applicable to each of the other disclosed embodiments. Thus, all combinations of the various elements described herein are within the scope of the invention. It should also be understood that, unless clearly indicated to the contrary, in any methods claimed herein that include more than one step or act, the order of the steps or acts of the method is not necessarily limited to the order in which the steps or acts of the method are recited.

### SEQUENCE LISTING

Sequence ID NO 1:
   *> Ricordea florida* HH (CORAL)-SARS RNA precursor DNA sequence (HH ribozyme from *Ricordea florida* genome assembly, chromosome: 1, GenBank ID OX453272.1)
   T7 promoter in bold, 5' HH ribozyme sequence underlined. 
TAATACGACTCACTATAGGCTATCAGCACTGCACTGATGAGGCCCAGAAGGCCGAAACAG
TACTGTCTGCAGTTAGATATGGTATTATGTTATTGTTTGTTCTGGGCTTAAAGAAGTACCATT
AGGTGGTATCGTGTTGTTGTTTATTCTAGATTTAAAGACCTGGGGGCATCTGGTACTGTGTT
GTCGTCTATTTTAGACTTAAAGTGGGCGATCGAGTTGGTACCATGTCATCGTTTATTTTAGG
CTTAAACACAGATTCGCCTCTGGTACTATGTCGCCGTTTATTTTGGGTTTAAATATGCGTGG
AGCAATGGTATCATGTTACCGTCTATTTTGAACTTAAA
Sequence ID NO 2:
   *> Trichobilharzia regenti* HH (TRE)-SARS RNA precursor DNA sequence (HH ribozyme from *Trichobilharzia regenti* genome assembly, scaffold: TRE_scaffold0011296, GenBank ID LL011296.1)
   T7 promoter in **bold,** 5' HH ribozyme sequence underlined. 
TAATACGACTCACTATAGGCGTACTGCTGAAGAGTCCCAAACTAGGACGAAACAACCAATT
GTCCAGTACTGGTATTATGTTATTGTTTGTTCTGGGCTTAAAGAAGTACCATTAGGTGGTAT
CGTGTTGTTGTTTATTCTAGATTTAAAGACCTGGGGGCATCTGGTACTGTGTTGTCGTCTAT
TTTAGACTTAAAGTGGGCGATCGAGTTGGTACCATGTCATCGTTTATTTTAGGCTTAAACAC
AGATTCGCCTCTGGTACTATGTCGCCGTTTATTTTGGGTTTAAATATGCGTGGAGCAATGGT
ATCATGTTACCGTCTATTTTGAACTTAAA
Sequence ID NO 3:
   *>* ***Schistosoma rodhaini* HH (SROB)-SARS RNA precursor DNA sequence** (HH ribozyme from *Schistosoma rodhaini* strain *Burundi* genome assembly, scaffold: SROB_scaffold0017863, GenBank ID LL975185.1)
   T7 promoter in **bold,** 5' HH ribozyme sequence underlined. 
TAATACGACTCACTATAGGCATCCAGCTGAAGAGTCCAAAATAGGACGAAGTGCCGCGCA
TCCTGGATTGGTATTATGTTATTGTTTGTTCTGGGCTTAAAGAAGTACCATTAGGTGGTATC
GTGTTGTTGTTTATTCTAGATTTAAAGACCTGGGGGCATCTGGTACTGTGTTGTCGTCTATT
TTAGACTTAAAGTGGGCGATCGAGTTGGTACCATGTCATCGTTTATTTTAGGCTTAAACACA
GATTCGCCTCTGGTACTATGTCGCCGTTTATTTTGGGTTTAAATATGCGTGGAGCAATGGTA
TCATGTTACCGTCTATTTTGAACTTAAA
Sequence ID NO 4:
   *>* ***Schistosoma rodhaini* HH extended (SROB2)-SARS RNA precursor DNA sequence** (HH ribozyme from *Schistosoma rodhaini* strain *Burundi* genome assembly, scaffold: SROB_scaffold0017863, GenBank ID LL975185.1)
   T7 promoter in bold, 5' HH ribozyme sequence underlined. 
TAATACGACTCACTATAGGCAGGTACATCCAGCTGAAGAGTCCAAAATAGGACGAAGTGC
CGCGCATCCTGGATTTCACTGCTGGTATTATGTTATTGTTTGTTCTGGGCTTAAAGAAGTAC
CATTAGGTGGTATCGTGTTGTTGTTTATTCTAGATTTAAAGACCTGGGGGCATCTGGTACTG
TGTTGTCGTCTATTTTAGACTTAAAGTGGGCGATCGAGTTGGTACCATGTCATCGTTTATTT
TAGGCTTAAACACAGATTCGCCTCTGGTACTATGTCGCCGTTTATTTTGGGTTTAAATATGC
GTGGAGCAATGGTATCATGTTACCGTCTATTTTGAACTTAAA
Sequence ID NO 5:
   > **Artificial HH 1 (ART1)-SARS RNA precursor DNA sequence**
   T7 promoter in **bold,** 5' HH self-catalytic ribozyme sequence underlined. 
TAATACGACTCACTATAGGCAGAGGTCTCCGGGCTGAAGAGGCGCAAAACGCCGAAACC
GATAGGTCCCCGGTTCCTCATGGTATTATGTTATTGTTTGTTCTGGGCTTAAAGAAGTACCA
TTAGGTGGTATCGTGTTGTTGTTTATTCTAGATTTAAAGACCTGGGGGCATCTGGTACTGTG
TTGTCGTCTATTTTAGACTTAAAGTGGGCGATCGAGTTGGTACCATGTCATCGTTTATTTTA
GGCTTAAACACAGATTCGCCTCTGGTACTATGTCGCCGTTTATTTTGGGTTTAAATATGCGT
GGAGCAATGGTATCATGTTACCGTCTATTTTGAACTTAAA
Sequence ID NO 6:
   > **Artificial HH 2 (ART2)-SARS RNA precursor DNA sequence**
   T7 promoter in bold, 5' HH self-catalytic ribozyme sequence underlined. 
TAATACGACTCACTATAGGCAGCGGTCTCCGGCTGAAGAGGCGCAAAACGCCGAAACC
GATAGGTCCCCGGTTCCGCATGGTATTATGTTATTGTTTGTTCTGGGCTTAAAGAAGTACCA
TTAGGTGGTATCGTGTTGTTGTTTATTCTAGATTTAAAGACCTGGGGGCATCTGGTACTGTG
TTGTCGTCTATTTTAGACTTAAAGTGGGCGATCGAGTTGGTACCATGTCATCGTTTATTTTA
GGCTTAAACACAGATTCGCCTCTGGTACTATGTCGCCGTTTATTTTGGGTTTAAATATGCGT
GGAGCAATGGTATCATGTTACCGTCTATTTTGAACTTAAA
Sequence ID NO 7:
   > **Artificial HH 3 (ART3)-SARS RNA precursor DNA sequence**
   T7 promoter in bold, 5' HH self-catalytic ribozyme sequence underlined. 
TAATACGACTCACTATAGGCACAGGTCTCCGGGCTGAAGAGGCGCAAAACGCCGAAACC
GATAGGTCCCCGGTTCCTGATGGTATTATGTTATTGTTTGTTCTGGGCTTAAAGAAGTACCA
 
TTAGGTGGTATCGTGTTGTTGTTTATTCTAGATTTAAAGACCTGGGGGCATCTGGTACTGTG
TTGTCGTCTATTTTAGACTTAAAGTGGGCGATCGAGTTGGTACCATGTCATCGTTTATTTTA
GGCTTAAACACAGATTCGCCTCTGGTACTATGTCGCCGTTTATTTTGGGTTTAAATATGCGT
GGAGCAATGGTATCATGTTACCGTCTATTTTGAACTTAAA
Sequence ID NO 8:
   > **PCR forward primer containing EcoRI restriction site**
   EcoRI restriction site sequence underlined
      TTTGAATTCGTAATACGACTCACTATAGGC
Sequence ID NO 9:
   > **PCR reverse primer containing BamHl restriction site**
   BamHl restriction site sequence underlined
      TTTGGATCCGTTTAAGTTCAAAATAGACGG
Sequence ID NO 10:
   > **Forward primer for sequencing (M13 forward primer)**
   GTAAAACGACGGCCAG
Sequence ID NO 11:
   > **Reverse primer for sequencing (M13 reverse primer)**
   CAGGAAACAGCTATGAC
Sequence ID NO 12:
   *>* ***Ricordea florida* HH (CORAL)-SARS-ELVd circRNA precursor DNA sequence**
   T7 promoter in bold, 5' HH ribozyme sequence underlined, 3' HH ELVd ribozyme sequence in *italics.* 
TAATACGACTCACTATAGGCTATCAGCACTGCACTGATGAGGCCCAGAAGGCCGAAACAG
TACTGTCTGCAGTTAGATATGGTATTATGTTATTGTTTGTTCTGGGCTTAAAGAAGTACCATT
AGGTGGTATCGTGTTGTTGTTTATTCTAGATTTAAAGACCTGGGGGCATCTGGTACTGTGTT
GTCGTCTATTTTAGACTTAAAGTGGGCGATCGAGTTGGTACCATGTCATCGTTTATTTTAGG
CTTAAACACAGATTCGCCTCTGGTACTATGTCGCCGTTTATTTTGGGTTTAAATATGCGTGG
AGCAATGGTATCATGTTACCGTCTATTTTGAACTTAAAGACAGTCGGGTGGTGTGTGCCAC
CCCTGATGAGACCGAAAGGTCGAAACTGTC
Sequence ID NO 13:
   *>* ***Trichobilharzia regenti* HH (TRE)-SARS-ELVd circRNA precursor DNA sequence**
   T7 promoter in **bold,** 5' HH ribozyme sequence underlined, 3' HH ELVd ribozyme sequence in *italics.* 
TAATACGACTCACTATAGGCGTACTGCTGAAGAGTCCCAAACTAGGACGAAACAACCAATT
GTCCAGTACTGGTATTATGTTATTGTTTGTTCTGGGCTTAAAGAAGTACCATTAGGTGGTAT
CGTGTTGTTGTTTATTCTAGATTTAAAGACCTGGGGGCATCTGGTACTGTGTTGTCGTCTAT
TTTAGACTTAAAGTGGGCGATCGAGTTGGTACCATGTCATCGTTTATTTTAGGCTTAAACAC
AGATTCGCCTCTGGTACTATGTCGCCGTTTATTTTGGGTTTAAATATGCGTGGAGCAATGGT
ATCATGTTACCGTCTATTTTGAACTTAAAGACAGTCGGGTGGTGTGTGCCACCCCTGATGA
GACCGAAAGGTCGAAACTGTC
Sequence ID NO 14:
   *>* ***Schistosoma rodhaini* HH (SROB)-SARS-ELVd circRNA precursor DNA sequence**
   T7 promoter in bold, 5' HH ribozyme sequence underlined, 3' HH ELVd ribozyme sequence in *italics.* 
TAATACGACTCACTATAGGCATCCAGCTGAAGAGTCCAAAATAGGACGAAGTGCCGCGCA
TCCTGGATTGGTATTATGTTATTGTTTGTTCTGGGCTTAAAGAAGTACCATTAGGTGGTATC
GTGTTGTTGTTTATTCTAGATTTAAAGACCTGGGGGCATCTGGTACTGTGTTGTCGTCTATT
TTAGACTTAAAGTGGGCGATCGAGTTGGTACCATGTCATCGTTTATTTTAGGCTTAAACACA
GATTCGCCTCTGGTACTATGTCGCCGTTTATTTTGGGTTTAAATATGCGTGGAGCAATGGTA
TCATGTTACCGTCTATTTTGAACTTAAAGACAGTCGGGTGGTGTGTGCCACCCCTGATGAG
ACCGAAAGGTCGAAACTGTC
Sequence ID NO 15:
   *>* ***Schistosoma rodhaini* HH extended (SROB2)-SARS-ELVd circRNA precursor DNA sequence**
   T7 promoter in **bold,** 5' HH ribozyme sequence underlined, 3' HH ELVd ribozyme sequence in *italics.* 
TAATACGACTCACTATAGGCAGGTACATCCAGCTGAAGAGTCCAAAATAGGACGAAGTGC
CGCGCATCCTGGATTTCACTGCTGGTATTATGTTATTGTTTGTTCTGGGCTTAAAGAAGTAC
CATTAGGTGGTATCGTGTTGTTGTTTATTCTAGATTTAAAGACCTGGGGGCATCTGGTACTG
TGTTGTCGTCTATTTTAGACTTAAAGTGGGCGATCGAGTTGGTACCATGTCATCGTTTATTT
TAGGCTTAAACACAGATTCGCCTCTGGTACTATGTCGCCGTTTATTTTGGGTTTAAATATGC
GTGGAGCAATGGTATCATGTTACCGTCTATTTTGAACTTAAAGACAGTCGGGTGGTGTGTG
CCACCCCTGATGAGACCGAAAGGTCGAAACTGTC
Sequence ID NO 16:
   **> Artificial HH 1 (ART1)-SARS-ELVd circRNA precursor DNA sequence**
   T7 promoter in bold, 5' HH self-catalytic ribozyme sequence underlined, 3' HH ELVd ribozyme sequence in *italics.* 
TAATACGACTCACTATAGGCAGAGGTCTCCGGGCTGAAGAGGCGCAAAACGCCGAAACC
GATAGGTCCCCGGTTCCTCATGGTATTATGTTATTGTTTGTTCTGGGCTTAAAGAAGTACCA
TTAGGTGGTATCGTGTTGTTGTTTATTCTAGATTTAAAGACCTGGGGGCATCTGGTACTGTG
TTGTCGTCTATTTTAGACTTAAAGTGGGCGATCGAGTTGGTACCATGTCATCGTTTATTTTA
GGCTTAAACACAGATTCGCCTCTGGTACTATGTCGCCGTTTATTTTGGGTTTAAATATGCGT
GGAGCAATGGTATCATGTTACCGTCTATTTTGAACTTAAAGACAGTCGGGTGGTGTGTGCC
ACCCCTGATGAGACCGAAAGGTCGAAACTGTC
Sequence ID NO 17:
   > **Artificial HH 2 (ART2)-SARS-ELVd circRNA precursor DNA sequence**
   T7 promoter in bold, 5' HH self-catalytic ribozyme sequence underlined, 3' HH ELVd ribozyme sequence in *italics.* 
TAATACGACTCACTATAGGCAGCGGTCTCCGGCTGAAGAGGCGCAAAACGCCGAAACC
GATAGGTCCCCGGTTCCGCATGGTATTATGTTATTGTTTGTTCTGGGCTTAAAGAAGTACCA
TTAGGTGGTATCGTGTTGTTGTTTATTCTAGATTTAAAGACCTGGGGGCATCTGGTACTGTG
TTGTCGTCTATTTTAGACTTAAAGTGGGCGATCGAGTTGGTACCATGTCATCGTTTATTTTA
GGCTTAAACACAGATTCGCCTCTGGTACTATGTCGCCGTTTATTTTGGGTTTAAATATGCGT
GGAGCAATGGTATCATGTTACCGTCTATTTTGAACTTAAAGACAGTCGGGTGGTGTGTGCC
ACCCCTGATGAGACCGAAAGGTCGAAACTGTC
Sequence ID NO 18:
   > Artificial HH 3 (ART3)-SARS-ELVd circRNA precursor DNA sequence
   T7 promoter in bold, 5' HH self-catalytic ribozyme sequence underlined, 3' HH ELVd ribozyme sequence in *italics.* 
TAATACGACTCACTATAGGCACAGGTCTCCGGGCTGAAGAGGCGCAAAACGCCGAAACC
GATAGGTCCCCGGTTCCTGATGGTATTATGTTATTGTTTGTTCTGGGCTTAAAGAAGTACCA
TTAGGTGGTATCGTGTTGTTGTTTATTCTAGATTTAAAGACCTGGGGGCATCTGGTACTGTG
TTGTCGTCTATTTTAGACTTAAAGTGGGCGATCGAGTTGGTACCATGTCATCGTTTATTTTA
GGCTTAAACACAGATTCGCCTCTGGTACTATGTCGCCGTTTATTTTGGGTTTAAATATGCGT
GGAGCAATGGTATCATGTTACCGTCTATTTTGAACTTAAAGACAGTCGGGTGGTGTGTGCC
ACCCCTGATGAGACCGAAAGGTCGAAACTGTC
Sequence ID NO 19:
   > **PCR reverse primer containing BamHl restriction site for constructs containing** ELVd **ribozyme at the 3' region.**
   BamHl restriction site sequence underlined
      TTTGGATCCGGACAGTTTCGACCTTTCGGTCTC
Sequence ID NO 33: synthetic catalytic ribozyme/polyribonucleotide, general sequence: N₁N₂GGYCUCCGGGCUGAAGAGGCGCAAAACGCCGAAACC, wherein N₁, N₂, can be any nucleotide, provided that N₁ is capable of base pair with N₄ of SEQ ID NO: 20, and provided that N₂ is capable of base pair with N₃ of SEQ ID NO: 20.
Sequence ID NO 20: substrate polyribonucleotide, general sequence:
   GGUCCCCGGUHCCN₃N₄, wherein N₃, N₄, can be any nucleotide, provided that N₄ is capable of base pair with N₁ of SEQ ID NO: 33, and provided that N₃ is capable of base pair with N₂ of SEQ ID NO: 33.
Sequence ID NO 21: synthetic self-catalytic ribozyme/polyribonucleotide (cis cleavage): N₁N₂GGYCUCCGGGCUGAAGAGGCGCAAAACGCCGAAACCN₅GGUCCCCGGUHCCN₃N₄, wherein N₁, N₂, N₃, and N₄ can be any nucleotides, provided that N₁ is capable of base pair with N₄ and N₂ is capable of base pair with N₃, and wherein N₅ is a linker, preferably the nucleotide sequence "GAUA".
Sequence ID NO 22: synthetic self-catalytic ribozyme/polyribonucleotide (cis cleavage): N₁N₂GGYCUCCGGGCUGAAGAGGCGCAAAACGCCGAAACCGAUAGGUCCCCGGUHCCN₃N ₄ wherein N₁, N₂, N₃, and N₄ can be any nucleotides, provided that N₁ is capable of base pair with N₄ and N₂ is capable of base pair with N₃.
Sequence ID NO 23: synthetic catalytic ribozyme/polyribonucleotide HH1:
   GGCAGAGGUCUCCGGGCUGAAGAGGCGCAAAACGCCGAAACC
Sequence ID NO 24: substrate polyribonucleotide of synthetic catalytic ribozyme/polyribonucleotide HH1:
   GGUCCCCGGUUCCUCA
Sequence ID NO 25: synthetic catalytic ribozyme/polyribonucleotide HH2:
   GGCAGCGGUCUCCGGGCUGAAGAGGCGCAAAACGCCGAAACC
Sequence ID NO 26: substrate polyribonucleotide of synthetic catalytic ribozyme/polyribonucleotide HH2:
   GGUCCCCGGUUCCGCA
Sequence ID NO 27: synthetic catalytic ribozyme/polyribonucleotide HH3:
   GGCACAGGUCUCCGGGCUGAAGAGGCGCAAAACGCCGAAACC
Sequence ID NO 28: substrate polyribonucleotide of synthetic catalytic ribozyme/polyribonucleotide HH3:
   GGUCCCCGGUUCCUGA
Sequence ID NO 29: synthetic self-catalytic ribozyme/polyribonucleotide HH1 (cis cleavage): 
GGCAGAGGUCUCCGGGCUGAAGAGGCGCAAAACGCCGAAACCGAUAGGUCCCCGGUUC
CUCA
Sequence ID NO 30: synthetic self-catalytic ribozyme/polyribonucleotide HH2 (cis cleavage): 
GGCAGCGGUCUCCGGGCUGAAGAGGCGCAAAACGCCGAAACCGAUAGGUCCCCGGUUC
CGCA
Sequence ID NO 31: synthetic self-catalytic ribozyme/polyribonucleotide HH3 (cis cleavage): 
GGCACAGGUCUCCGGGCUGAAGAGGCGCAAAACGCCGAAACCGAUAGGUCCCCGGUUC
CUGA
Sequence ID NO 32: ELVd self-catalytic ribozyme
   GACAGTCGGGTGGTGTGTGCCACCCCTGATGAGACCGAAAGGTCGAAACTGTC

The invention is described below by the following examples, which must be considered as merely illustrative and in no case limiting of the scope of the present invention.

### EXAMPLES

### EXAMPLE 1:

In this example, we show the *in vitro* cleavage efficiency of 7 constructs, each with a different synthetic self-cleaving ribozyme in the 5' region. Here, we compare 4 previously known naturally occurring hammerhead ribozymes (from *Ricordea florida, Trichobilharzia regent and Schistosoma rodhaini* -2 sequences-) with three new artificial designed self-cleaving ribozymes.

The methodology to obtain the desired constructs and validate the self-cleaving ribozymes consisted of two steps:
1) Production of T7 promoter-5' ribozyme DNA templates (by cloning).
   First, the precursor RNA sequences preceded by a T7 promoter followed by the 5' self-cleaving ribozyme of interest (encoded by sequences **SEQ ID NO: 1-7)** obtained from Integrated DNA Technologies were amplified in a PCR reaction to introduce EcoRI and BamHl restriction sites using primers **SEQ ID NO: 8** and **SEQ ID NO: 9.** PCR products were purified with a Phenol:Chloroform:lsoamyl alcohol extraction and then double digested and cloned into purified BamHl-EcoRl linearized pUC18 plasmid using T4 DNA ligase (1:3, insertvector ratio). *E.coli* DH5α chemically competent cells were transformed with 5 µL of ligation product and single colonies were grown overnight at 37°C in the presence of 100 µg/mL ampicillin. The resulting plasmids were isolated and the DNA sequences were verified by sequencing (using M13 primers, **SEQ ID NO: 10** and **SEQ ID NO: 11).**
2) Self-processing of self-cleaving ribozyme-RNAs.
   After sequence verification, pUC18-RNA of interest constructs were linearized with Hindlll enzyme and purified with a Phenol:Chloroform:Isoamyl alcohol extraction. Purified DNAs were *in vitro* transcribed using T7 RNA Polymerase from Takara as detailed: 3 µg of digested plasmid were mixed with 10 mM DTT, 2 mM NTPs, 100 U RNAse OUT and 125 U T7 RNA Polymerase in 1X T7 RNA polymerase Reaction Buffer (40 mM Tris-HCl pH 8.0, 8 mM MgCl₂ and 2 mM spermidine) and incubated at 37°C for 3 h. The resultant RNAs produced were analyzed by denaturing polyacrylamide gel electrophoresis (Figure 1).

As shown in Figure 1, our three artificial 5'-HH self-cleaving ribozymes (encoded by **SEQ ID NO 5-7 corresponding to lanes 5-7)** designed showed an optimal self-cleavage resulting in a RNA fragment of approximately 300 nt. Previously described naturally occurring HH from *Ricordea florida* and *Trichobilharzia regenti* (encoded by **SEQ ID NO 1 and SEQ ID NO 2,** respectively, in lanes 1-2) are also self-cleaved forming the 300 nt RNA fragments whereas none of the two HH from *Schistosoma rodhaini* (encoded by **SEQ ID NO 3 and SEQ ID NO 4,** lanes 3-4) are self-cleaved. Some degradation of the in vitro transcription product from the *Trichobilharzia regenti* construct (encoded by **SEQ ID NO 2)** is observed.

### EXAMPLE 2:

In this example, we show the *in vitro* cleavage efficiency of 7 precursor circRNAs each flanked by one of the 5' HH self-cleaving ribozymes analyzed in example 1 (HH from *Ricordea florida, Trichobilharzia regenti, Schistosoma rodhaini* -2 sequences-, and our three new artificial designed ribozymes) and the HH ELVd ribozyme in the 3' region.

As in previous example 1, the methodology to obtain the desired precursor circRNAs and validate the self-cleaving ribozymes consisted of the following two steps:
1) Production of T7 promoter-self-cleaving ribozymes flanked DNA templates (by cloning). First, the precursor circRNA sequences preceded by a T7 promoter and flanked by the 5' self-cleaving ribozyme of interest and the 3' HH ELVd self-cleaving ribozyme (encoded by sequences **SEQ ID NO: 12-18)** obtained from Integrated DNA Technologies were amplified in a PCR reaction to introduce EcoRI and BamHl restriction sites using primers **SEQ ID NO: 8** and **SEQ ID NO: 19.** PCR products were purified with a Phenol:Chloroform:Isoamyl alcohol extraction and then double digested and cloned into purified BamHI-EcoRI linearized pUC18 plasmid using T4 DNA ligase (1:3, insert:vector ratio). *E.coli* DH5α chemically competent cells were transformed with 5 µL of ligation product and single colonies were grown overnight at 37°C in the presence of 100 µg/mL ampicillin. The resulting plasmids were isolated and the precursor circRNA DNA sequences were verified by sequencing (using M13 primers, **SEQ ID NO: 10** and **SEQ ID NO: 11).**
2) Self-processing of the self-cleaving ribozyme-RNAs.
   After sequence verification, pUC18-circRNA constructs were linearized with Hindlll enzyme and purified with a Phenol: Chloroform: Isoamyl alcohol extraction. Purified DNAs were *in vitro* transcribed using T7 RNA Polymerase from Takara as detailed: 3 µg of digested plasmid were mixed with 10 mM DTT, 2 mM NTPs, 100 U RNAse OUT and 125 U T7 RNA Polymerase in 1X T7 RNA polymerase Reaction Buffer (40 mM Tris-HCl pH 8.0, 8 mM MgCl₂ and 2 mM spermidine) and incubated at 37°C for 3 h. The resultant RNAs produced were analyzed by denaturing polyacrylamide gel electrophoresis (Figure 2).

As shown in Figure 2, the three precursor circRNA containing our three artificial 5'-HH self-cleaving ribozymes (encoded by **SEQ ID NO 16-18;** in lanes 5-7) designed showed a double self-cleavage resulting in a RNAfragment of approximately 300 nt. The construct containing previously described naturally occurring HH from *Ricordea florida* **(SEQ ID NO 12,** lane 1) is also double self-cleaved forming the 300 nt circRNA fragments, however, a less intense band is shown in the polyacrylamide-urea gel suggesting a lower self-cleavage efficiency. Although single self-cleavage by one of the two ribozymes is observed (bands B and C) in all constructs, negligible double self-cleavage by the two HH is observed in constructs that contain the 5' HH self-cleaving ribozymes from *Trichobilharzia regenti* (encoded by **SEQ ID NO 13,** lane 2) or *Schistosoma rodhaini* (encoded by **SEQ ID NO 14** and SEQ ID NO 15, lane 3-4).

### EXAMPLE 3:

In this example, we show the circularization efficiency of 7 precursor circRNAs from Example 2, each flanked by one of the 5' HH self-cleaving ribozymes analyzed in example 1 and the HH ELVd self-cleaving ribozyme in the 3' region.

The methodology to obtain the desired circRNAs and validate the circularization capacity of the self-cleaving ribozymes consisted of the following two steps:
1) RNA circularization and purification
   1 µg of linear RNA coming from Example 2 were circularized with RtcB ligase (New England Biolabs, M0458S) following manufacturer's instructions during 1 hour at 37°C. The circularized RNA was purified through a Chloroform/isoamyl-alcohol precipitation.
2) CircRNA selection
   The obtained circRNA was subjected to an RNAse R (Abcam, ab286929) reaction to eliminate the possible linear RNA remains, following manufacturer's instructions. To ensure the correct circRNA generation, 2 µl of the final circRNA samples were run in a 40% Acrylamide/Urea gel, 200 V for 2 hours. Gel was stained with ethidium bromide for 10 minutes and analyzed in a UV transilluminator (Bio Rad).

As shown in Figure 3, the three precursor circRNA containing our three artificially designed 5'-HH self-cleaving ribozymes (encoded by **SEQ ID NO 16-18,** lanes 5-7) showed a 100% circularization efficiency. CircRNA precursors containing previously described naturally occurring HH from *Ricordea florida* (encoded by **SEQ ID NO 12,** lane 1) *and Schistosoma rodhaini* (encoded by **SEQ ID NO 14,** lane 3), presented two bands corresponding to the generated circRNA but also the precursor linear RNA showing an incomplete circularization. For circRNA precursors containing previously described naturally occurring HH from *Trichobilharzia regenti* (encoded by **SEQ ID NO 13,** lane 2) and *Schistosoma rodhaini* (encoded by **SEQ ID NO 15,** lane 4), no circularization was observed.

## Claims

1. A **synthetic catalytic polyribonucleotide** comprising SEQ ID NO: 33, or a system of molecules that give rise to said synthetic catalytic polyribonucleotide,
wherein the catalytic polyribonucleotide is capable of exerting a catalytic cleavage activity on a substrate polyribonucleotide, wherein the substrate polyribonucleotide comprises the sequence of SEQ ID NO: 20, and wherein N₁, N₂ in SEQ ID NO: 33, and N₃, N₄ in SEQ ID NO: 20 can be any nucleotide, provided that N₁ is capable of base pair with N₄ and N₂ is capable to base pair with N₃.

2. The synthetic catalytic polyribonucleotide according to claim 1, wherein:
a) the polyribonucleotide comprises or consists of SEQ ID NO: 23, wherein the polyribonucleotide is capable of exerting a catalytic cleavage activity on a substrate polyribonucleotide, wherein the substrate polyribonucleotide preferably comprises the sequence of SEQ ID NO: 24;
b) the polyribonucleotide comprises or consists of SEQ ID NO: 25, wherein the polyribonucleotide is capable of exerting a catalytic cleavage activity on a substrate polyribonucleotide, wherein the substrate polyribonucleotide preferably comprises the sequence of SEQ ID NO: 26; or
c) the polyribonucleotide comprises or consists of SEQ ID NO: 27, wherein the polyribonucleotide is capable of exerting a catalytic cleavage activity on a substrate polyribonucleotide, wherein the substrate polyribonucleotide preferably comprises the sequence of SEQ ID NO: 28.

3. The synthetic catalytic polyribonucleotide according to claims 1 or 2, wherein the substrate polyribonucleotide is comprised in the transcriptome of an organism and wherein the catalytic polyribonucleotide is not comprised in the transcriptome of said organism, so that the catalytic polyribonucleotide exerts a *trans* catalytic cleavage activity on said substrate polyribonucleotide.

4. A **synthetic self-catalytic polyribonucleotide,** or a system of molecules that give rise to said synthetic self-catalytic catalytic polyribonucleotide, comprising at least two regions that are covalently connected by a linker, wherein a first region comprises the synthetic catalytic polyribonucleotide as defined in any of claims 1 to 3, wherein a second region comprises the substrate polyribonucleotide of said synthetic catalytic polyribonucleotide, and wherein the catalytic polyribonucleotide of the first region exerts a *cis* catalytic cleavage activity on the substrate polyribonucleotide of the second region.

5. The synthetic self-catalytic polyribonucleotide according to claim 4, comprising any of the following sequences:
a) SEQ ID NO: 21, wherein N₁, N₂, N₃, and N₄ can be any nucleotides, provided that N₁ is capable of base pair with N₄ and N₂ is capable of base pair with N₃, and wherein N₅ is a linker, preferably the nucleotide sequence "GAUA";
b) SEQ ID NO: 22, wherein N₁, N₂, N₃, and N₄ can be any nucleotides, provided that N₁ is complementary to N₄ and N₂ is complementary to N₃;
c) SEQ ID NO: 29;
d) SEQ ID NO: 30; or
e) SEQ ID NO: 31.

6. A **DNA molecule** comprising at least one sequence that, upon transcription, generates an RNA molecule comprising a hammerhead type I ribozyme in its 5' end and a hammerhead type III ribozyme in its 3' end.

7. A **DNA molecule** comprising at least one sequence that, upon transcription, generates an RNA molecule comprising the synthetic catalytic polyribonucleotide of any one of claims 1 to 3, or the synthetic self-catalytic polyribonucleotide of any of claims 4 or 5.

8. The DNA molecule according to claim 7, wherein the DNA molecule comprises the synthetic self-catalytic polyribonucleotide of any of claims 4 or 5 in its 5' end.

9. A **composition** comprising the catalytic polyribonucleotide defined in any of claims 1 to 3, the synthetic self-catalytic polyribonucleotide of any of claims 4 or 5, or the DNA molecule as defined in any of claims 6 to 8.

10. The catalytic polyribonucleotide as defined in any of claims 1 to 3, the synthetic self-catalytic polyribonucleotide of any of claims 4 or 5, the DNA molecule as defined in any of claims 6 to 8, or the composition as defined in claim 9, **for use** in medicine.

11. A **method** to provide a plurality of RNA molecules **characterized by** having a sequence in the 5' end that is identical or substantially identical in all of the molecules, the method comprising the steps of:
a) Providing a plurality of DNA molecules **characterized in that**, when transcribed, result in a plurality of RNA molecules, each of said RNA molecules being **characterized by** comprising the synthetic self-catalytic polyribonucleotide as defined in any one of claims 4 or 5 located in the 5' end, and
b) *In vitro* or *in vivo* transcribing the plurality DNA molecules of a), thereby producing a plurality of RNA molecules that are self-cleaved by action of the synthetic self-catalytic polyribonucleotide.

12. A **method** to produce a circular RNA molecule, the method comprising the steps of:
a) Providing a DNA molecule **characterized in that**, when transcribed, results in an RNA molecule comprising a first and a second regions, wherein:
i. the first region comprises the synthetic self-catalytic polyribonucleotide as defined in any one of claims 4 or 5, and
ii. the second region comprises:
1. a self-catalytic polyribonucleotide that, when cleaved, generates an end capable of being ligated to the end generated after the self-cleavage of the synthetic self-catalytic polyribonucleotide of the first region, or
2. a sequence capable of being ligated to the end generated after the self-cleavage of the synthetic self-catalytic polyribonucleotide of the first region,
b) *in vitro* or *in vivo* transcribing the DNA molecule of step a), thereby producing an RNA molecule that is self-cleaved by action of the synthetic self-catalytic polyribonucleotide of the first region and of the self-catalytic polyribonucleotide of the second region, if present, so that a RNA molecule with ligatable ends is obtained, and
c) adding a ligase capable of joining the 5' and 3' end of the cleaved RNA molecule obtained in step b), to provide a circular RNA molecule.

13. The method according to claim 12, wherein the first region is located in the 5' end of the RNA molecule, and the second region is located in the 3' end of the RNA molecule.

14. A **circular RNA molecule** obtained or obtainable from the method defined in any of claims 12 or 13.

15. A **method** to produce a circular RNA molecule, the method comprising the steps of:
a) providing a DNA molecule **characterized in that**, when transcribed, results in an RNA molecule comprising a hammerhead type I ribozyme in its 5' end and a hammerhead type III ribozyme in its 3' end,
b) *in vitro* or *in vivo* transcribing the DNA molecule of step a), thereby producing an RNA molecule that is self-cleaved by action of the hammerhead type I and II ribozymes, so that a RNA molecule with ligatable ends is obtained, and
c) adding a ligase capable of joining the 5' and 3' end of the cleaved RNA molecule obtained in step b), to provide a circular RNA molecule.
